(19) Europäisches Patentamt
European Patent Office
Office européen des brevets

(11) EP 0 863 767 B1

(12) EUROPÄISCHE PATENTSCHRIFT

(45) Veröffentlichungstag und Bekanntmachung des Hinweises auf die Patenterteilung:
**17.04.2002 Patentblatt 2002/16**

(21) Anmeldenummer: **96939084.8**

(22) Anmeldetag: **18.11.1996**

(51) Int Cl.[7]: **A61K 47/48**, C08F 8/18, A01N 59/12, A61P 1/12

(86) Internationale Anmeldenummer:
**PCT/EP96/05062**

(87) Internationale Veröffentlichungsnummer:
**WO 97/19702 (05.06.1997 Gazette 1997/24)**

(54) **PULVERFÖRMIGE IOD-KOMPLEXE**

POWDERY IODINE COMPLEXES

COMPLEXES D'IODE EN POUDRE

(84) Benannte Vertragsstaaten:
**AT BE CH DE DK ES FI FR GB IT LI NL SE**

(30) Priorität: **29.11.1995 DE 19544449**

(43) Veröffentlichungstag der Anmeldung:
**16.09.1998 Patentblatt 1998/38**

(73) Patentinhaber: **BASF AKTIENGESELLSCHAFT
67056 Ludwigshafen (DE)**

(72) Erfinder:
- **BREITENBACH, Jörg
D-68199 Mannheim (DE)**
- **SANNER, Axel
D-67227 Frankenthal (DE)**
- **DENZINGER, Walter
D-67346 Speyer (DE)**
- **LANG, Siegfried
D-67071 Ludwigshafen (DE)**

(56) Entgegenhaltungen:
**DE-A- 2 437 629 DE-A- 4 414 254
GB-A- 1 166 766**

- **CHEMICAL ABSTRACTS, vol. 73, no. 26, 28.Dezember 1970 Columbus, Ohio, US; abstract no. 131624, CRAWSHAW A. ET AL.: "Preparation and polymerization of N,N'-Divinylureas. Polymerization studies and spectroscopic investigation of structure" XP002032171 & J. MACROMOL. SCI., CHEM. (1971), A9(7), 1903-1914 CODEN: JMCHBD, 1971,**
- **CHEMICAL ABSTRACTS, vol. 99, no. 22, 28.November 1983 Columbus, Ohio, US; abstract no. 176411, KIRSH, YU. E. ET AL: "Poly(N-vinylamides), complexation and conformational changes in aqueous solution" XP002032172 & EUR. POLYM. J. (1983), 19(7), 639-45 CODEN: EUPJAG;ISSN: 0014-3057, 1983,**
- **J. MACROMOL. SCI., CHEM. (1975), A9(7), 1085-111 CODEN: JMCHBD, 1975, XP002032170 CORFIELD, G. C. ET AL: "N,N'-Divinylureas. Polymerization studies and spectroscopic investigation of structure"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 863 767 B1

Printed by Jouve, 75001 PARIS (FR)

## Beschreibung

Die vorliegende Erfindung betrifft pulverförmige Komplexe aus Iod und einem vernetzten Polymerisat auf Basis von N-Vinylverbindungen, erhältlich durch trockenes Erhitzen von Iod und einem Polymerisat, welches durch Polymerisation von Monovinylverbindungen (Monomere A), deren Vinylgruppe an ein N-Atom eines stickstoffhaltigen Heterocyclus gebunden ist, in Gegenwart von 0,5 bis 10 Gew.-%, bezogen auf die Monomeren (A), einer Verbindung (B) der allgemeinen Formel I

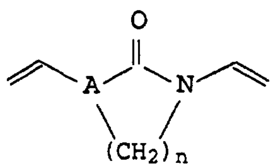

I

worin A für -CH- oder ein N-Atom steht und n = 2 oder 3 ist, erhalten wird.

Weiterhin betrifft die Erfindung ein Verfahren zur Herstellung solcher Komplexe und deren Verwendung zur Herstellung von Medikamenten gegen Durchfallerkrankungen.

Es ist bekannt, daß vernetztes genau wie lösliches PVP mit Iod Komplexe bildet. Das Iod ist an den unlöslichen Träger fester gebunden als an den löslichen. Trotzdem ist die Menge an freiem Iod in Kontakt mit Feuchtigkeit, z.B. Wundsekret und anderen Körperflüssigkeiten, ausreichend, um Mikroorganismen abzutöten (vgl. z.B. WO 92/04031).

Verfahren zur Herstellung von vernetztem PVP-Iod sind beispielsweise aus US 3 136 755 und US 3 907 720 bekannt. Jedoch führen beide Verfahren nicht zu stabilen, einheitlichen, frei fließenden Pulvern. In US 5 152 987 wird zwar die Herstellung eines vernetzten PVP-Iods in der Form von frei fließenden Pulvern beschrieben, doch wird dabei Isopropanol als Lösungsmittel eingesetzt. Dies kann zu iodierten Nebenprodukten führen, ganz abgesehen von den medizinischen, ökologischen und ökonomischen Nachteilen der Anwendung organischer Lösungsmittel.

FR 2 353 297 beschreibt zwar die Herstellung von vernetztem PVP-Iod in Form von frei fließenden Pulvern durch direkte Umsetzung von PVP und Iod, jedoch sind zusätzliche Verfahrensschritte erforderlich, wie die Reinigung des Polymeren durch Behandlung mit siedendem Wasser und Ethanol und anschließendes Trocknen im Vakuum, so daß das Verfahren für die Praxis wenig geeignet ist.

Der Erfindung lag die Aufgabe zugrunde, freifließende pulverförmige Romplexe aus Iod und Polymerisaten auf Basis von N-Vinyllactamen zur Verfügung zu stellen, die auf einfache Weise erhältlich sind.

Demgemäß wurden die eingangs definierten Iod-Komplexe, ein Verfahren zur Herstellung und deren Verwendung gefunden.

Als Monovinylverbindungen (Monomere A) eignen sich Stickstoffheterocyclen, die an einem Ringstickstoffatom mit einer Vinylgruppe substituiert sind, geeignete Monomere (A) sind vor allem ausgewählt aus der Gruppe bestehend aus N-Vinyllactamen, N-Vinylimidazol und N-Vinylcarbazol. Als N-Vinyllactame kommen 5-, 6- oder 7-gliedrige Lactame in Betracht, die am Ring auch Methyl-, Ethyloder Propyl-Substituenten tragen können. Bevorzugte N-Vinyllactame sind N-Vinylpyrrolidon und N-Vinylcaprolactam.

Die erfindungsgemäB verwendeten Polymerisate können auch aus Gemischen der oben genannten Monomeren erhalten werden, beispielsweise durch Copolymerisation von 5 bis 80 Gew.-% N-Vinylpyrrolidon und 20 bis 95 Gew.-% N-Vinylcaprolactam.

Weiterhin eignen sich erfindungsgemäß auch Copolymerisate, die neben den Monomeren (A) noch bis zu 80 Gew.-% weiterer monoolefinisch ungesättigter Monomere enthalten, vor allem Vinylester wie Vinylacetat, Vinylpropionat oder Vinylbutyrat, sowie auch Acrylsäure und/oder Methacrylsäure und deren $C_1$-$C_4$-Alkylester.

Besonders bevorzugte Polymerisate enthalten als Monomere (A) N-Vinylpyrrolidon oder Mischungen aus N-Vinylpyrrolidon und N-Vinylcaprolactam.

Die Monomeren (A) werden in Gegenwart von vernetzend wirkenden Verbindungen (B) der allgemeinen Formel I polymerisiert. Als Verbindungen (B) kommen vor allem cyclische Amide, die neben einer N-Vinylgruppe eine weitere Vinylgruppe tragen, in Betracht. Geeignete Verbindungen sind beispielsweise cyclische N,N'-Divinylalkylenharnstoffe wie N,N'-Divinylethylenharnstoff oder N,N'-Divinyl-propylenharnstoff. Weiterhin eignen sich auch N,N'-Divinyl-imidazolidon-(2) oder N-Vinyl-3-ethyliden-pyrrolidon-(2) oder N-Vinyl-3-ethyliden-piperidinon-(2).

Die Herstellung der Polymerisate kann nach an sich bekannten Verfahren, bevorzugt durch Popcorn- Polymerisation, erfolgen.

Ein geeignetes Verfahren ist beispielsweise in der DE-PS 20 59 484 beschrieben, wobei die Polymerisation in wäßrigem Medium in Gegenwart von Eisen, einer durch Sauerstoff angreifbaren Eisenlegierung, Kobalt, Zink oder

Zinn erfolgt.

Weiterhin eignet sich auch das in der DE-OS 22 55 263 beschriebene Verfahren, wobei die Polymerisation in wäßriger Lösung durch Erhitzen auf 80°C gestartet und bei der Siedetemperatur des Wassers zu Ende geführt wird.

Ebenso eignet sich auch das aus der DE-PS 2437640 bekannte Verfahren der Polymerisation unter Sauerstoffausschluß in Gegenwart einer α- oder β-Ketocarbonsäure oder eines entsprechenden Methyloder Ethylesters.

Bevorzugt können die Polymerisate nach dem in der DE-PS 24 37 629 beschriebenen Verfahren hergestellt werden, wobei die Polymerisation unter Sauerstoffausschluß in Gegenwart von 0,05 bis 2 Gew.-% einer Schwefelverbindung mit einer Wertigkeit unter 6 durchgeführt wird. Geeignete Schwefelverbindungen werden ausgewählt aus der Gruppe der Sulfite, Pyrosulfite, Dithionite, der Sulfoxylate und der Sulfide, wobei bevorzugt die entsprechenden Natriumsalze eingesetzt werden.

Die geschilderten Verfahren können in Abwesenheit üblicher Radikalspender in wäßriger Lösung durchgeführt werden.

Die erfindungsgemäß zur Herstellung der Komplexe eingesetzten Polymerisate sind im wesentlichen unlöslich, d.h. sie enthalten weniger als 2 Gew.-% lösliche Anteile. Bevorzugte Polymerisate weisen spezifische Oberflächen (BET) im Bereich von 0,5 - 5 $m^2/g$, besonders bevorzugt 0,7 - 4 $m^2/g$ auf.

Zur Herstellung der erfindungsgemäßen Iod-Komplexe werden die Komponenten, also das elementare Iod und das Polymerisat, in Abwesenheit eines Lösungsmittels trocken intensiv gemischt und auf Temperaturen von 50 bis 150°C, vorzugsweise 70 bis 120°C, erhitzt. Das Mischen und Erhitzen erfolgt üblicherweise in einem Doppelkonusmischer, kann aber auch in Schaufelradmischern, Schneckenmischern oder Luftmischern durchgeführt werden. Die Dauer des Erhitzens kann sich unter anderem nach der Größe des Ansatzes richten oder nach dem angestrebten Iod-Gehalt, wobei der Fachmann diese auf einfache Weise entsprechend ermitteln kann.

In den nachstehenden Beispielen wurde der frei verfügbare Iodgehalt auf folgende Weise bestimmt.

Bestimmung des verfügbaren Iodgehaltes:

Ca. 2 g unlöslicher Iodkomplex werden in einem 250 ml Erlenmeyerkolben mit 100 ml destilliertem Wasser suspendiert, mit Eisessig sauer gestellt und mit 25 ml 0,1 n Natriumthiosulfatlösung versetzt. Nun wird ca. 1 h geschüttelt, bis die Suspension völlig entfärbt ist. dann wird über ein Faltenfilter abfiltriert, mit ca. 50 ml destilliertem Wasser nachgewaschen und mit 0,1 n Iod-Kaliumiodidlösung gegen Stärke bis zur Blaufärbung titriert.

Berechnung:

$$\frac{\text{(ml Vorlage an } Na_2S_2O_3 \text{ ./. Verbrauch an Iod) x 127}}{\text{Einwaage x 100}} = \text{\% verfügbares Iod}$$

Bestimmung des Verteilungskoeffizienten (VK) :

1 g einer Suspension des unlöslichen Iodkomplexes mit 1 % verfügbarem Iod wird in einem 50 ml Meßkolben mit 25 ml n-Heptan bei 25°C 10 min. intensiv geschüttelt. Nach ca. zweiminütigem Stehen, wenn Phasentrennung eingetreten ist, wird die Heptanphase abfiltriert und deren Iodgehalt photometrisch bestimmt. Der Iodgehalt der wäßrigen Phase wird aus der Differenz des eingesetzten Iodgehaltes minus dem Iodgehalt in der Heptanphase errechnet.

Die erfindungsgemäß erhältlichen Iodkomplexe sind lagerbeständig (kein Iodverlust), einheitlich (keine Konzentrationsschwankungen) und für praktische Zwecke frei von Nebenprodukten, das Pulver fließt frei, neigt beim Lagern nicht zum Verbacken, hat eine spezifische Oberfläche (BET) von 0,5 $m^2/g$ bis 5 $m^2/g$, vorzugsweise 0,9 bis 4 $m^2/g$ und hat einen Gehalt an verfügbarem Iod von 0,5 bis 18, vorzugsweise 8 bis 13 Gew.-%. Sie haben bei der Anwendung den großen Vorteil, daß das Iod einerseits so fest gebunden ist, daß auch die orale Applikation ohne Komplikationen hinsichtlich der Schilddrüse möglich ist, andererseits doch so viel Iod freigesetzt wird, daß Krankheitskeime (Bakterien) und Viren zuverlässig abgetötet werden. Daher ist die orale Anwendung gegen Diarrhoe Teil der Erfindung. Das Produkt wirkt zweifach: es bindet einerseits Flüssigkeit unter Quellung und tötet andererseits die Erreger ab.

Beispiele 1 - 11 In den nachstehenden Beispielen erfolgte die Vermischung der Komponenten in einem Doppelkonus-Mischer.

Verwendete Abkürzungen:

| | |
|---|---|
| VP | N-Vinylpyrrolidon |
| VCAP | N-Vinylcaprolactam |

Die in den nachstehenden Beispielen verwendeten Polymere wurden wie in Beispiel 1 der DE-OS 2437629 beschrieben hergestellt und durch Mahlung auf die gewünschte spezifische Oberfläche (BET) (Bestimmung nach DIN 66131-132) eingestellt.

Polymer I:

unlösliches PVP, BET = 1,5 - 2 $m^2/g$

Polymer II:

unlösliches PVP, BET = 0,9 $m^2/g$

Polymer III:

unlösliches PVP, BET = 0,7 - 0,9 $m^2/g$

Polymer IV:

Polymerisat, erhalten durch Copolymerisation von VP und VCAP im Gewichtsverhältnis 1:5 analog Beispiel 1 der DE-OS 2437629, BET = 1,3 $m^2/g$

Polymer V:

Polymerisat, erhalten durch Copolymerisation von VP und VCAP im Gewichtsverhältnis 1:1, BET = 0,7 $m^2/g$

3. 249 g Polymer 1, 51 g Iod wurden 2 h bei 70°C und 24 h bei 100°C gemischt.

| | |
|---|---|
| Feststoffgehalt | 97.7 Gew.-% |
| verfügbarer Iodgehalt | 9.95 Gew.-% |
| Verteilungskoeffizient | 187 |

4. 451 g Polymer II, 85 g Iod wurden 2 h bei 70°C und 24 h bei 100°C gemischt.

| | |
|---|---|
| Feststoffgehalt | 96.9 Gew. -% |
| verfügbarer Iodgehalt | 9.32 Gew.-% |
| Verteilungskoeffizient | 220 |

5. 60 kg Polymer III, 12.3 kg Iod wurden 1 h bei Raumtemperatur, 2 h bei 70°C und 20 h bei 100°C gemischt.

| | |
|---|---|
| Feststoffgehalt | 97.0 Gew.-% |
| verfügbarer Iodgehalt | 12.0 Gew.-% |
| Verteilungskoeffizient | 173 |

6. 200 g Polymer III, 41 g Iod wurden 1 h bei Raumtemperatur, 2 h bei 70°C und 10 h bei 100°C gemischt.

| | |
|---|---|
| Feststoffgehalt | 97.5 Gew.-% |
| verfügbarer Iodgehalt | 10.3 Gew.-% |
| Verteilungskoeffizient | 293 |

7. 451 g Polymer IV, 85 g Iod wurden 2 h bei 70°C und 24 h bei 100°C gemischt.

| | |
|---|---|
| Feststoffgehalt | 94.96 Gew.-% |
| verfügbarer Iodgehalt | 7.4 Gew.-% |
| Verteilungskoeffizient | 779 |

8. 451 g Polymer V, 85 g Iod wurden 2 h bei 70°C und 24 h bei 100°C gemischt.

| | |
|---|---|
| Feststoffgehalt | 91.5 Gew.-% |
| verfügbarer Iodgehalt | 10.9 Gew.-% |
| Verteilungskoeffizient | 249,8 |

9. 451 g Polymer V, 85 g Iod wurden 2 h bei 70°C und 16 h bei 120°C gemischt.

| | |
|---|---|
| Feststoffgehalt | 90.5 Gew.-% |
| verfügbarer Iodgehalt | 10.5 Gew.-% |
| Verteilungskoeffizient | 200,8 |

10. 300 kg Polymer II, 60 kg Iod wurden 1 h bei Raumtemperatur, 2 h bei 70°C und 30 h bei 95°C gemischt.

| | |
|---|---|
| Feststoffgehalt | 97.1 Gew.-% |
| verfügbarer Iodgehalt | 11.02 Gew.-% |
| Verteilungskoeffizient | 220 |

11. 60 g Polymer III, 12.3 kg Iod wurden 1 h bei Raumtemperatur, 2 h bei 70°C und 10 h bei 105°C gemischt.

| | |
|---|---|
| Feststoffgehalt | 97.0 Gew.-% |
| verfügbarer Iodgehalt | 11.6 Gew.-% |
| Verteilungskoeffizient | 171 |

12. 60 kg Polymer III, 12.3 kg Iod wurden 1 h bei Raumtemperatur, 2 h bei 70°C und 20 h bei 105°C gemischt.

| | |
|---|---|
| Feststoffgehalt | 97.0 Gew.-% |
| verfügbarer Iodgehalt | 11.3 Gew.-% |
| Verteilungskoeffizient | 311 |

13. 60 kg Polymer III, 12.3 kg Iod wurden 1 h bei Raumtemperatur, 2 h bei 70°C und 20 h bei 115°C gemischt.

| | |
|---|---|
| Feststoffgehalt | 97.0 Gew.-% |
| verfügbarer Iodgehalt | 13.3 Gew.-% |
| Verteilungskoeffizient | 290 |

Formulierung von Antidiarrhoika

Beispiel 12

| | |
|---|---|
| Komplex gemäß Beispiel 1 | 50 mg |
| mikrokristalline Cellulose | 5 mg |
| Magnesiumstearat | 0,5 mg |

Beispiel 13

| | |
|---|---|
| Komplex gemäß Beispiel 2 | 200 mg |
| unlösliches PVP | 1800 mg |
| mikrokristalline Cellulose | 100 mg |

(fortgesetzt)

| | |
|---|---|
| Kieselsäure | 100 mg |
| Magnesiumstearat | 10 mg |

Die Formulierungen gemäß den Beispielen 12 und 13 eignen sich zur Direkttablettierung.

Beispiel 14

Eine Mischung aus 200 mg eines Komplexes gemäß Beispiel 2 und 1800 mg unlöslichem PVP wurden mit 20 mg Povidon K 90 und 9,1 g Ethanol einer Feuchtgranulierung unterworfen. Nach dem Trocknen der Granalien wurden 10 mg Magnesiumstearat zugesetzt. Die Formulierung kann unter herkömmlichen Bedingungen tablettiert werden.

## Patentansprüche

1. Pulverförmige Komplexe aus Iod und einem vernetzten Polymerisat auf Basis von N-vinylverbindungen, erhältlich durch trokkenes Erhitzen von Iod und einem Polymerisat, welches durch Polymerisation von Monovinylverbindungen (Monomere A), deren Vinylgruppe an ein N-Atom eines stickstoffhaltigen Heterocyclus gebunden ist, in Gegenwart von 0,5 bis 10 Gew.-%, bezogen auf die Monomeren (A), einer Verbindung (B) der allgemeinen Formel I

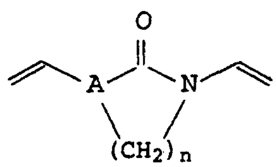

I

worin A für -CH- oder ein N-Atom steht und n = 2 oder 3 ist, erhalten wird.

2. Verwendung von Komplexen gemäß Anspruch 1 zur Herstellung eines Antidiarrhoikums.

## Claims

1. A complex in powder form of iodine and a crosslinked polymer based on N-vinyl compounds, obtainable by dry heating of iodine and a polymer which is obtained by polymerization of monovinyl compounds (monomers A) whose vinyl group is bonded to a nitrogen atom of a nitrogen-containing heterocycle, in the presence of from 0.5 to 10% by weight, based on the monomers (A), of a compound (B) of the formula I

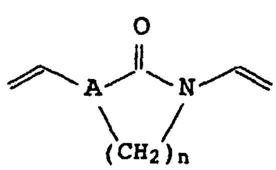

I

where A is -CH- or a nitrogen atom, and n is 2 or 3.

2. The use of a complex as claimed in claim 1 for producing an antidiarrheal.

## Revendications

1. Complexes pulvérulents consistant en iode et un polymère réticulé à base de dérivés N-vinyliques, obtenus par chauffage à sec de l'iode et d'un polymère lui-même obtenu par polymérisation de dérivés monovinyliques (monomères A) dont le groupe vinyle est fixé sur un atome d'azote d'un hétérocycle azoté, en présence de 0,5 à 10 % en poids, par rapport aux monomères A, d'un composé B de formule générale I

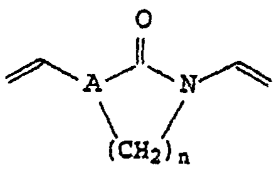

I

dans laquelle A représente -CH- ou un atome d'azote et n = 2 ou 3.

2. Utilisation des complexes selon la revendication 1 pour la préparation d'un antidiarrhéique.